# EUROPEAN PATENT APPLICATION

(11) **EP 1 947 169 A1**
(43) Date of publication of application: **23.07.2008**
(21) Application number: 07100767.8
(22) Date of filing: 18.01.2007
(51) Int. Cl.: C12M 1/24, C12M 3/04

(54) **Method of filling a flask**

(71) Applicant: The Automation Partnership (Cambridge) Limited, Royston, Hertfordshire SG8 5WY (GB)
(72) Inventor: Golding, Matthew Steven, Chesterton, Cambridgeshire CB4 1QD (GB)
(74) Representative: Smee, Anthony James Michael

(57) **Abstract**

A method of filling a flask, the flask having a plurality of individual liquid flaskettes, the flaskettes each being provided with at least one opening into an inlet chamber, the chamber being defined by one end of each of the individual flaskettes and an inlet boundary wall, the inlet boundary wall having at least one inlet through which, in use, a liquid media can be introduced into the flask, the flask also having a longitudinal axis parallel to the planes of the individual flaskettes, the method comprising the step of presenting the flask to a liquid media supply nozzle at a predetermined orientation such that liquid flows from the nozzle along the inlet boundary wall until it can pass directly into one or more of the individual flaskettes.

## Description

This invention relates to a method of filling a flask and also to an apparatus for use when filling a flask, the flask typically being a multilayered high density cell culture vessel.

At present, it is well known to grow cells in a culture vessel and there are two types of cells which can be grown. "Adherent cells" preferentially form on a surface, whereas "suspension cells" grow within a liquid suspension. The present invention is directed to flasks for growing adherent cells.

Initially, adherent cells were grown in flasks which had a single layer or surface on which the cells could grow. Subsequently, in order to make better use of the space within a flask, a triple layered flask was designed. More recently, a further improvement on this is known in which a plurality, typically 10, flaskettes are joined together and supplied by a common inlet. Thus the flask has a plurality of individual flaskettes, the flaskettes each being provided with at least one opening into an inlet chamber, the chamber being defined by one end of each of the individual flaskettes and an inlet boundary wall, the inlet boundary wall having at least one inlet through which, in use, a liquid media can be introduced into the flask. This construction maximises the surface area, and hence the number of cells which can be grown in a given volume.

Each flaskette comprises a tray section having an open side and a semi-permeable membrane covering the open side of the tray, thereby defining a chamber into which the liquid media is supplied and the cells grow.

However, this presents a problem when filling the flask as the media added into the flask typically includes proteins, often supplied as bovine serum, as well as other nutrients necessary for cell growth. This liquid media is inherently "foamy", by which we mean that, when disturbed, the media readily produces lots of tiny air bubbles as the liquid is disturbed, and this results in unwanted foaming of the liquid media within the flask. Given the surface tension of the liquid media due to the protein material, the bubbles do not readily collapse, so the foam does not dissipate. Such foaming reduces the volume available for the liquid media itself and, in order to ensure the flask is filled as full as possible with nutrients and that no or little unwanted air is present, it is necessary to remove the foam, either by dislodging the air bubbles or by physically removing the foamed material through the neck of the flask. Such a procedure is time consuming and costly as it wastes the liquid media which had been added and which had foamed. In addition, the presence of bubbles in the liquid media is detrimental to cell growth and cell viability.

The problem of foaming occurs whether the flask has one or multiple layers on which the cells are to be grown, but it is a particular problem when using a multilayered vessel, i.e. a collection of flaskettes, as the liquid media entering the flask typically impacts upon the ends of the individual flaskettes, within which one or more openings have been provided in order to allow liquid to flow into the individual flaskettes. Such impact on the ends of the flaskettes inherently disturbs the flow of the liquid media and increases the likelihood and occurrence of foaming. In addition to this, as liquid is caused to flow down the individual flaskettes along the smooth, relatively large surfaces on which the cells are to be grown, the liquid flow tends to become chaotic and this also generates bubbles within the liquid media.

Whilst it is well known from pouring any form of liquid media which tends to foam, for example, carbonated drinks such as cola, lemonade or champagne, that the pouring should be done such that the liquid does not impact on the bottom of the glass, but rather runs down the side of the glass, and that the pouring should be carried out at a relatively slow rate, thereby helping to minimise the number of bubbles which are formed, there is no specific method that eliminates this problem.

Whilst such a method does have some benefit when pouring liquid media into the flasks described above, it does not entirely solve the problem.

The present invention aims to overcome the problems described above and to provide an improved method of filling a flask.

According to the present invention, there is provided a method of filling a flask, the flask having a plurality of individual liquid flaskettes, the flaskettes each being provided with at least one opening into an inlet chamber, the chamber being defined by one end of each of the individual flaskettes and an inlet boundary wall, the inlet boundary wall having at least one inlet through which, in use, a liquid media can be introduced into the flask, the flask having a longitudinal axis parallel to the planes of the individual flaskettes, the method comprising the step of:
presenting the flask to a liquid media supply nozzle at a predetermined orientation such that liquid flows from the nozzle along the inlet boundary wall until it can pass directly into one or more of the individual flaskettes.

By ensuring that the orientation at which the flask is presented is such that liquid flows along the lower surface of the upper portion of the flask, i.e. the inlet boundary wall, until it can pass directly into the flaskettes, impacting the liquid on the ends of the individual flaskettes is minimised, thereby reducing the likelihood of foaming.

The flask typically has a rectangular cross section perpendicular to its longitudinal axis and, in the predetermined orientation, the flow is preferably directed along the inlet boundary wall to the shorter edge of the rectangular cross section of the flask and, preferably, to one of the corners of that cross section, i.e. to the upper end and the side of one of the outermost flaskettes. This may be achieved by providing the inlet boundary wall with one or more shoulders, having on its inner surface an angle of less than 180°, such that flow along the shoulder is provided with some restriction to its flow in a sideways direction, i.e. the flow is preferentially directed along the shoulder. The one or more shoulders may be directed to the corners of the rectangular cross section.

From the shorter edge of the rectangular cross section, the flow is preferably directed down one of the longitudinal edges extending from each corner such that, again, the flow is constrained to flow along that internal edge and is not permitted to flow in a chaotic manner along one of the faces of the flask or the individual flaskettes within that flask.

The method preferably comprises the step of temporarily halting supply of the liquid into the flask and then returning the flask to a substantially vertical orientation, with the inlet opening uppermost. Having done this, additional liquid can be added into the flask in order to substantially fully fill the flask with the desired media. However, it is particularly advantageous that, after returning the flask to a substantially vertical orientation, the liquid in the flask completely fills the individual flaskettes, such that any additional liquid which is introduced into the flask does not impact on the ends of the individual flaskettes, but rather simply drops into the liquid which is already in the flask.

From a substantially vertical orientation with the longitudinal axis of the flask substantially vertical, the flask can preferably be tilted prior to filling with liquid such that the longitudinal axis is no longer vertical. The centre point of any of the rotations is the centre of the opening of the inlet. The first direction in which the flask may be tilted is such that the angle of a plane perpendicular to the flaskettes and passing through the longitudinal axis is between 32° and 58°, more preferably 42°, away from the vertical.

The flask is preferably presented to a liquid supply media nozzle such that the flow from the nozzle is between the centre of the opening and the upper (when tilted) portion of the rim of the neck. This ensures that, during supply of the liquid media, the media cannot overflow the lower (when tilted) portion of the rim of the nozzle.

The second direction in which the flask can be moved is rotation about its longitudinal axis away from a neutral position (i.e. one in which the lowermost edge of the flask is horizontal) between -10° and +20°, more preferably +5°. The third possible movement of the flask is tilting it such that a plane parallel to the individual flaskettes is between -50° and +72° away from the vertical, but preferably it is at 0°.

The flask is preferably tilted in the first direction, with the second direction providing further advantages. Rotation in the third direction has little impact on the filling of the flask, as can be seen by the preferred angle being 0°, except that tilting outside of the range given adversely affects the volume that can be supplied to the flask before having to make the flask more upright.

The flow rate at which the liquid media is supplied is important and is preferably between 2.5 ml/s and 5 ml/s per second, but preferably is as near to 5ml/s as possible, as this will ensure that the speed of filling is practical. Of course, the slower the pour, the less agitation of the liquid media is caused, but this can lead to the time taken for filling the flask being too slow and, as such, there is a practical lower limit to the speed at which flow can enter the flask. There is also a practical upper limit, driven by the need for the liquid to remain on the underside of the inlet boundary wall. This value is in the region of 5 ml/s, as anything above this value tends to see liquid dropping off the neck onto the individual flaskettes, rather than flowing on the underside of the inlet boundary wall. This will, however, be dependent upon the viscosity and surface tension of the liquid media to be added to the flask.

The present invention also provides an apparatus for holding a flask during filling, the flasks having a plurality of individual liquid flaskettes, the flaskettes each being provided with at least one opening into an inlet chamber, the chamber being defined by one end of each of the individual flaskettes and an inlet boundary wall, the inlet boundary wall having at least one inlet through which, in use, a liquid media can be introduced into the flask, the flask having a longitudinal axis parallel to the planes of the individual flaskettes, the apparatus comprising:
a holder shaped so as to support the flask, the holder being shaped and/or angled so as to present the flask at a predetermined angle suitable for filling the flask by liquid flowing from the nozzle along the inlet boundary wall until it can pass directly into one or more of the individual flaskettes.

The holder may be movable between two positions, the first of which holds the flask at the predetermined angle for filling, and the second of which holds the flask in a substantially vertical orientation. The holder may be movable to discreet positions, one at the predetermined angle and one at the substantially vertical orientation, or may be continuously variably movable between those extremes. Alternatively, the holder may be provided with a number of intermediate discreet positions between the two extremes at which the flask can be held.

The apparatus may also comprise a pump for controlling the speed of liquid supplied to the flask when the holder is in use.

Examples of the present invention will now be described with reference to the accompanying drawings, in which:
Figure 1 shows a schematic plan view of one example of a flask;
Figure 2 shows a sectional view along the longitudinal axis of the flask of Figure 1;
Figure 3 shows a view from the inlet end of the flask;
Figure 4 shows a perspective view of the flask indicating the different axes about which the flask can be moved in order to obtain the desired orientation;
Figure 5 shows a perspective view of an example of an apparatus for holding the flask during filling, with the flask angled at a predetermined orientation;
Figure 6 shows a different view of the apparatus of Figure 5, indicating one angle though which the flask has been tilted;
Figure 7 shows a further view of the apparatus of Figure 5, indicating rotation of the flask about its longitudinal axis;
Figure 8 shows the apparatus of Figure 5 with the flask in the substantially vertical orientation.

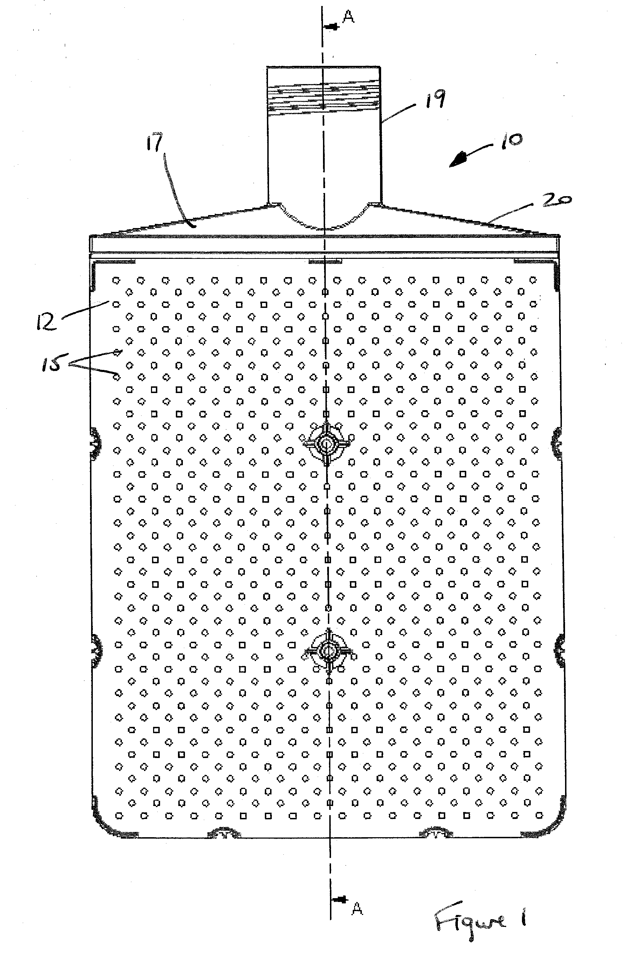

Figures 1 to 3 show an example of a flask which can be filled using the method of the present invention. The flask 10 comprises ten individual flaskettes 11, each of which comprises a tray section 12 having an open side and a semi-permeable membrane 13 covering the openside of the tray. A compartment 14 is defined between the tray and the semi-permeable membrane into which, in use, the liquid media is supplied and the cells grow. The underside of tray layer 12 is provided with a plurality of small projections 15 which, when adjacent flaskettes are brought together, rest against the semi-permeable membrane 13 of the adjacent flaskette and define an air gap which allows gas exchange with compartment 14. Oxygen can pass into the compartment and other waste gases such as carbon dioxide can pass out of the compartment 14 and be removed.

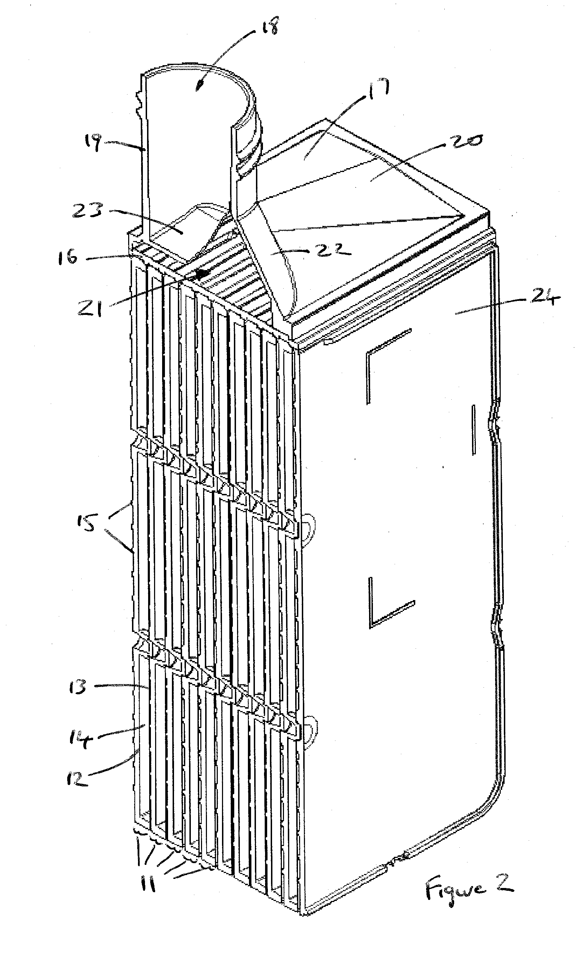

In Figures 1 and 2, the upper end of each of the individual flaskettes is engaged with a grating 16 which is provided with a plurality of openings (not shown), which allow liquid to flow through corresponding openings in the upper ends of the trays 12. Connected to the grating 16 is the inlet boundary wall 17 which defines an inlet 18 having a neck 19, and an intermediate surface 20 which joins the neck 19 to the grating 16 and the upper ends of trays 12. Thus, an inlet chamber 21 is defined between the inlet boundary wall 17, the grating 16 and the upper ends of trays 12.

The neck 19 is provided with a bulge 22 which is shaped to permit ready inflow of air into the flask when emptying the contents of the flask. The neck 19 is also provided with a dam 23 which, when the flask is in a horizontal orientation on bottom surface 24, prevents any gas trapped in the neck from passing into the individual flaskettes.

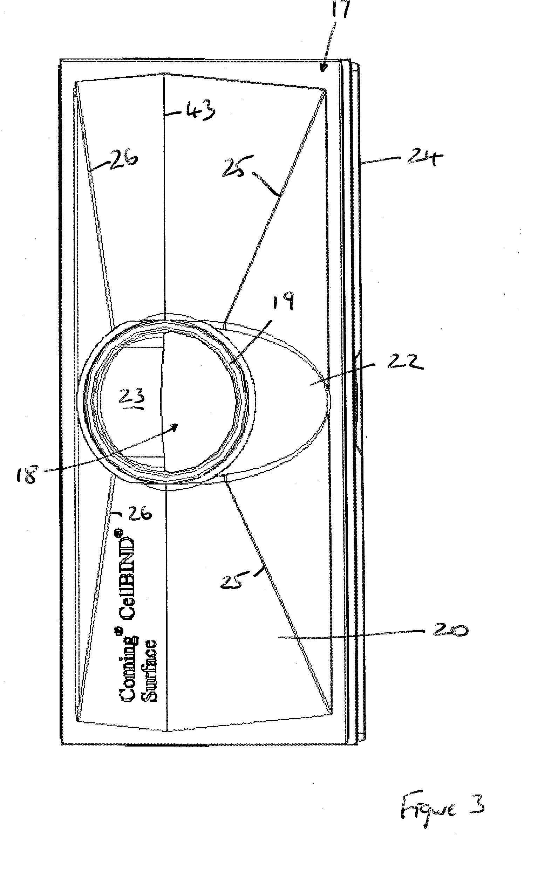

The inlet boundary wall 17 is shaped, as can be seen in the Figures, such that it slopes, when the flask is in the vertical orientation, downwardly from the lower portion of the neck 19 to the outer edge of the flask. As can be seen most clearly in Figures 2 and 3, the surface 20 is provided with a pair of shoulders 25, 26 on each side of the neck 19, these shoulders each having an internal angle of less than 180°.

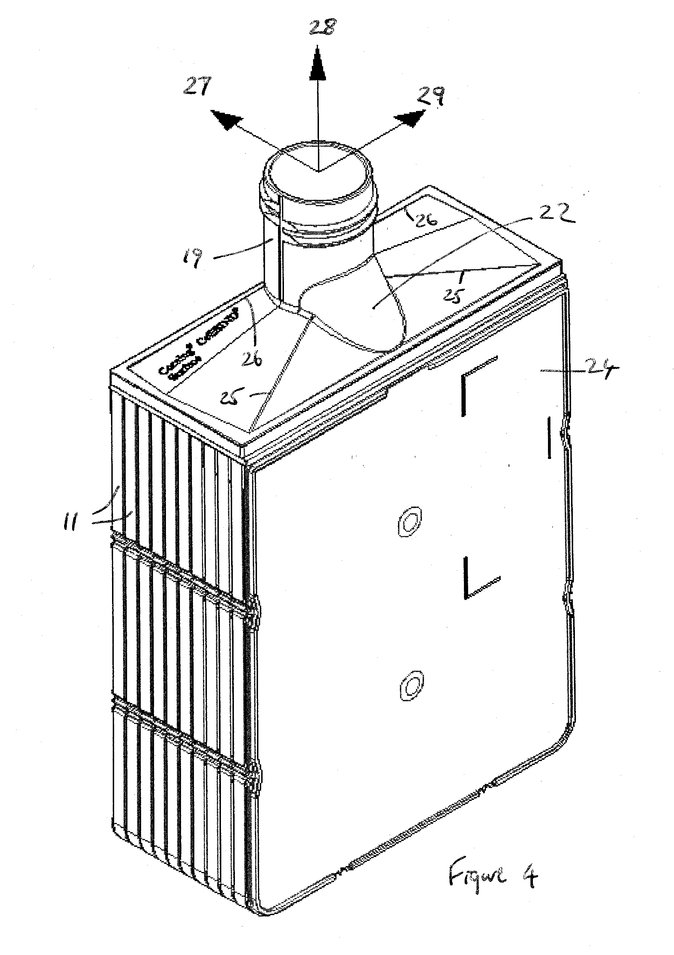

Figure 4 shows the three axes about which the flask can be rotated, in use, in order to obtain the desired orientation for filling. The origin of these three axes is the centre of the opening of the neck, but this is selected simply for ease of explaining the movement of the flask and could be located in any other suitable position to allow the desired end position of the flask to be obtained. Further description of the rotation and tilting of the flask with respect to these axes is described with reference to the later figures.

Typically, flasks of the sort described above are filled either by hand, i.e. by a user holding the flask at some undetermined and inherently variable angle due to the vagaries of the user holding the flask whilst pouring the desired liquid media into the inlet, or, alternatively, the flask can be manipulated by a robotic arm in a machine, such that the robotic arm can present the flask to one or more of a plurality of nozzles for supply of the desired liquid media, the robotic arm being programmed to hold the flask at a particular orientation.

It is, however, important to be able to fill the flask, either by hand or using a robot, as there may be certain situations in which one method is more preferrable to the other.

As such, it would be advantageous to have a simple apparatus for holding the flask at the most appropriate angle for pouring to thereby reduce the possibility of foaming. Whilst the following description relates to the use of such a desk top apparatus, the angles at which the flask is being held and rotated through are equally applicable to the mounting of the flask in a robot.

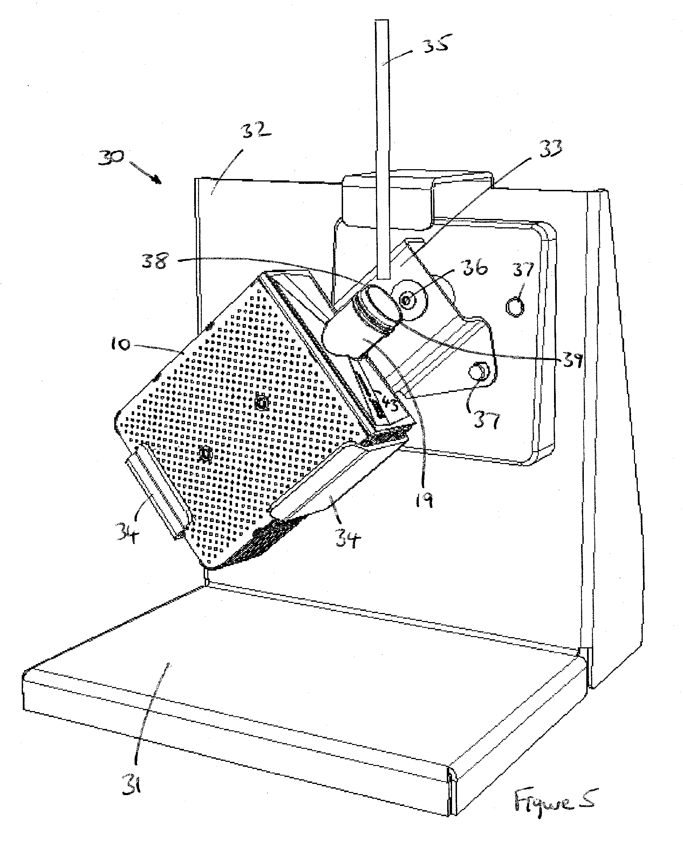

In Figure 5, a simple support apparatus 30 can be seen having a base 31 and a substantially vertical support section 32. A pivotable arm 33 is mounted to the support surface 32, the support arm 33 being provided with a plurality of retaining plates 34 for securely supporting a flask 10. The apparatus is provided with an inlet nozzle 35 which can be connected (not shown) to a supply of the desired liquid media. In addition, (again not shown), a pump may be located on the apparatus, typically on the reverse side of the support element 32, which can control the rate at which liquid is supplied through nozzle 35.

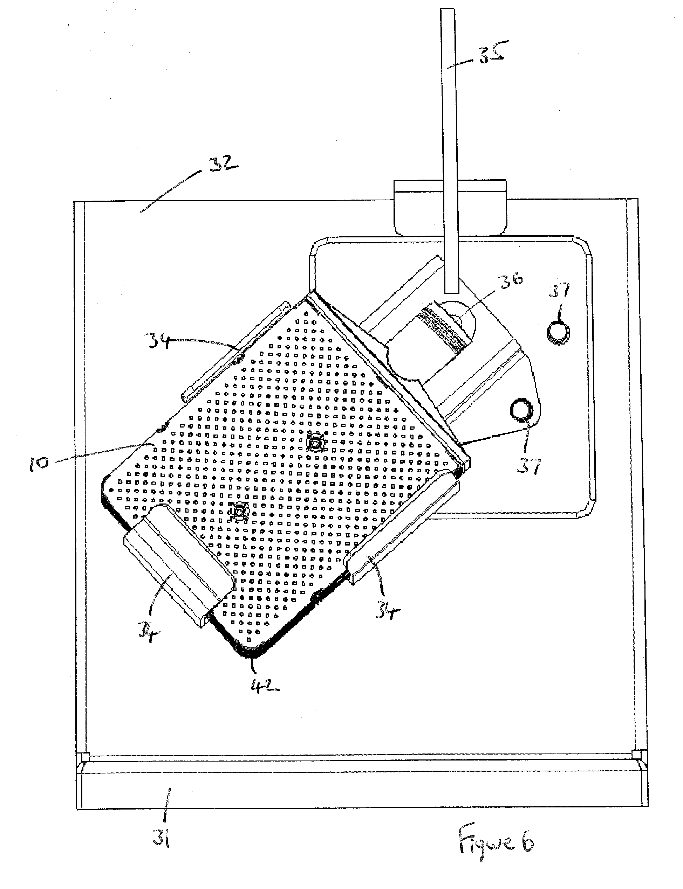

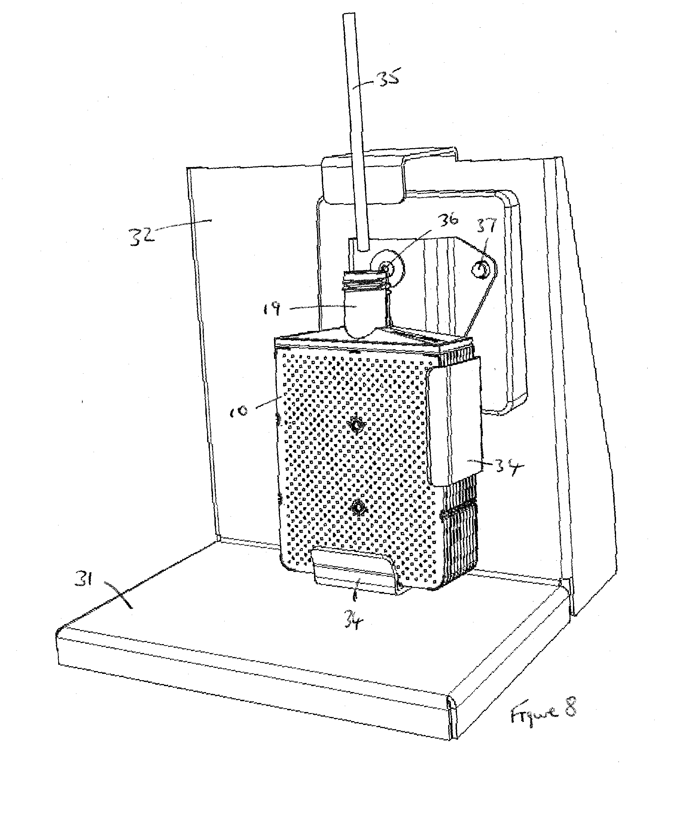

In the example shown, the movable arm 33 can be pivoted about pivot 36 (which for all practical purposes lies on axis 27 of Figure 4) such that, as can be seen in Figure 6, the flask is tilted with respect to the vertical. This is rotation about axis 27 shown in Figure 4. In this example, the flask is movable between two discreet positions defined by fixing locations 37 such that the flask can have the angled position shown in Figure 6, or the substantially vertical position shown in Figure 8.

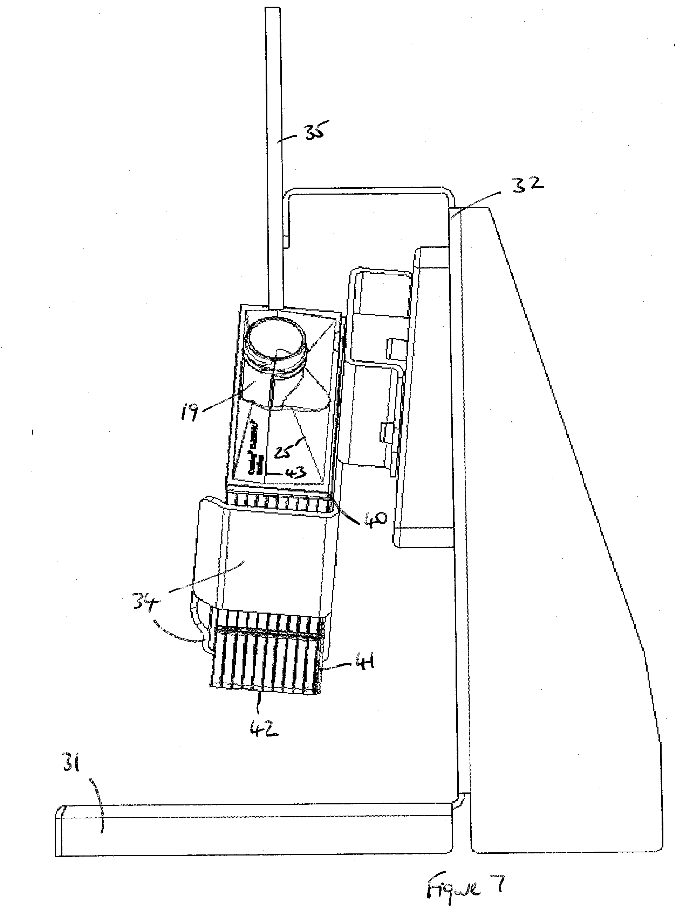

In addition, as can be seen in Figure 7, the flask has been rotated about its longitudinal axis, i.e. axis 28 as shown in Figure 4 away from a neutral position in which lowermost edge 42 is horizontal and, although the apparatus shown does not permit this rotation to be varied, as the arm is shaped such that this is the angle at which the flask is held, the apparatus may be adapted such that the angle could be varied. This is, of course, certainly true with a robotic arm. With reference to Figure 3, the neutral position is one in which line 43 is in a vertical plane. +20° of rotation would be intended to bring shoulder 25 into the vertical plane and -10° would bring shoulder 26 into the vertical plane. In Figure 7, the flask is shown at an angle of +5°.

The apparatus shown in Figures 5 to 8 does not permit rotation about axis 29 from Figure 4, but such rotation would, with reference to Figure 6, be rotation of the flask into and out of the page. +72° would be rotation out of the page and -50° would be into the page. In Figure 7, the flask has not been rotated about axis 29 and hence is at an angle of 0°.

When filling the flask, the flask is held as shown in Figure 5 and the inlet nozzle supplies liquid between the centre point of the opening of the neck 19 and the upper edge 38 of the flask to ensure that liquid does not spill over lower edge 39. The flow is preferably between 2.5 and 5ml/s and the liquid media is caused to flow under gravity, along the neck 19 until it reaches the intersection of the neck 19 with surface 20. The flow then, due to its surface tension, passes around the corner and passes along the underside of surface 20. Assuming the angles are selected appropriately, the flow will preferentially run along shoulder 25 until it reaches corner 40. The flow will then proceed down edge 41 thereby filling the individual flaskettes sequentially and in a controlled manner. As the liquid fills one flaskette, it will simply move on to the next.

Each flaskette holds typically 50ml and, if the angles described above are selected, approximately 510 ml can be placed into the flask without overflowing at the neck. The flask can then be moved to a second, preferably substantially vertical, orientation and the remaining space in the flask filled. Before the final "topping-up", it will be noted that the level of the liquid will be above the top of each flaskette (i.e. 510ml is greater than the volume of all 10 flaskettes), and therefore the additional liquid does not drop onto the grating 16 or the ends of trays 12, but rather drops into the bulk liquid which helps to minimise foaming. Typically, each flask is filled with 550 ml of liquid media.

Movement of the holder and therefore the flask could be carried out by hand or by using an actuator such as a motor.

## Claims

1. A method of filling a flask, the flask having a plurality of individual liquid flaskettes, the flaskettes each being provided with at least one opening into an inlet chamber, the chamber being defined by one end of each of the individual flaskettes and an inlet boundary wall, the inlet boundary wall having at least one inlet through which, in use, a liquid media can be introduced into the flask, the flask also having a longitudinal axis parallel to the planes of the individual flaskettes, the method comprising the step of:
presenting the flask to a liquid media supply nozzle at a predetermined orientation such that liquid flows from the nozzle along the inlet boundary wall until it can pass directly into one or more of the individual flaskettes.

2. A method according to claim 1, further comprising the step of supplying the liquid media at a predetermined flow rate.

3. A method according to any one of the preceding claims, further comprising the steps of:
temporarily halting supply of the liquid; and
returning the flask to a substantially vertical orientation with the inlet opening uppermost.

4. A method according to any one of the preceding claims, wherein the liquid flows along the inlet boundary wall to the smaller edge of the boundary before entering one or more of the individual flaskettes.

5. A method according to claim 4, wherein the flow is directed along a shoulder to a corner of the flask.

6. A method according to any one of the preceding claims, wherein, from a vertical orientation with the longitudinal axis substantially vertical, the flask can be tilted such that the longitudinal axis is no longer substantially vertical.

7. A method according to claim 6, wherein the flask can be tilted such that the angle of a plane perpendicular to the flaskettes and passing through the longitudinal axis is between 32° and 58°, more preferably 42°, away from the vertical.

8. A method according to claim 6 or claim 7, wherein the flask can be rotated about the longitudinal axis from a neutral position between -10° and +20°, more preferably +5°.

9. A method according to any one of claims 6 to 8, wherein the flask may be tilted such that a plane parallel to the individual flaskettes is between -50° and +72° away from the vertical, more preferably at 0°.

10. A method according to claim 9, wherein the flask is tilted and/or rotated in one or more of the three directions.

11. A method according to claim 10, wherein, when returned to the initial orientation, the liquid in the flask at least completely fills the individual flaskettes.

12. A method according to any one of the preceding claims, further comprising the step of supplying further liquid media to the flask in order to fill the space above the individual flaskettes bounded by the inlet boundary wall.

13. A method according to any one of claims 2 to 13, wherein the predetermined flow rate is between 2.5 and 5 ml/s, more preferably 5 ml/s.

14. An apparatus for holding a flask during filling, the flask having a plurality of individual liquid flaskettes, the flaskettes each being provided with at least one opening into an inlet chamber, the chamber being defined by one end of each of the individual flaskettes and an inlet boundary wall, the inlet boundary wall having at least one inlet through which, in use, a liquid media can be introduced into the flask, the flask having a longitudinal axis parallel to the planes of the individual flaskettes, the apparatus comprising:
a holder shaped so as to support the flask, the holder being shaped and/or angled so as to present the flask at a predetermined angle suitable for filling the flask by liquid flowing from the nozzle along the inlet boundary wall until it can pass directly into one or more of the individual flaskettes.

15. An apparatus according to claim 14, wherein the holder is movable between two positions, the first of which holds the flask at the predetermined angle, and the second of which holds the flask in a substantially vertical orientation.

16. An apparatus according to claim 15, wherein the holder is movable between two discreet positions.

17. An apparatus according to either claim 15 or claim 16, wherein the holder is provided with a one or more discreet positions between the two extremes at which it can be held.

18. An apparatus according to either claim 15 or claim 16, wherein the holder is continuously movable between the two extreme positions.

19. An apparatus according to any one of claims 14 to 18, further comprising a pump for use in controlling the flow of liquid into the flask.
